# EUROPÄISCHE PATENTSCHRIFT

(11) **EP 1 191 011 B1**
(45) Veröffentlichungstag und Bekanntmachung des Hinweises auf die Patenterteilung: **23.02.2005**
(21) Anmeldenummer: 01121345.1
(22) Anmeldetag: 06.09.2001
(51) Int. Cl.: C07C 41/09

(54) **Verfahren zur Herstellung von mittel-und langkettigen Dialkylethern**
Process for the preparation of medium and long chain dialkyl ethers
Procédé de préparation de dialkyléthers à moyennes et longues chaînes

(30) Priorität: 08.09.2000 DE 10044581; 08.09.2000 DE 20015578 U
(43) Veröffentlichungstag der Anmeldung: 27.03.2002
(73) Patentinhaber: DHW Deutsche Hydrierwerke GmbH Rodleben, 06862 Rodleben (DE)
(72) Erfinder: Schröter, Jörg., D-06862 Rosslau (DE); Weidemann, Frank., D-06862 Rosslau (DE); Konetzke, Gerhard, D-06847 Dessau (DE)
(74) Vertreter: Tragsdorf, Bodo, Dipl.-Ing.

(56) Entgegenhaltungen:
- DE-A- 4 127 230
- DE-A- 19 511 668
- DE-U- 20 015 578
- DATABASE WPI Section Ch, Week 199010 Derwent Publications Ltd., London, GB; Class B01, AN 1990-071803 XP002184393 & JP 02 025496 A (MITSUBISHI KASEI CORP), 26. Januar 1990 (1990-01-26)
- DATABASE WPI Section Ch, Week 198736 Derwent Publications Ltd., London, GB; Class B03, AN 1987-253880 XP002184394 & JP 62 175440 A (DAICEL CHEM IND LTD), 1. August 1987 (1987-08-01)
- DATABASE CA [Online] CHEMICAL ABSTRACTS SERVICE, COLUMBUS, OHIO, US; SUZUKI ET AL.: "Stereospecific construction of medium-sized cyclic ethers and its applications to synthesis of marine natural products" Database accession no. 131:73484 XP002184392 & TENNEN YUKI KAGOBUTSU TORONKAI KOEN YOSHISHU, Bd. 39th, 1997, Seiten 91-96,

## Beschreibung

Die Erfindung betrifft ein Verfahren zur Herstellung von mittel- und langkettigen Dialkylethern, bei dem man lineare und/oder verzweigte, gesättigte und/oder ungesättigte, ein- und/oder mehrwertige Alkohole mit 6 bis 22 Kohlenstoffatomen bei erhöhter Temperatur in Gegenwart eines Katalysators dehydratislert, das entstehende Reaktionswasser kontinuierlich abtrennt und das Rohprodukt nach der Katalysatorabtrennung in an sich bekannter Weise reinigt.

Dialkylether finden auf Grund ihrer speziellen chemisch-physikalischen Eigenschaften ein breites Anwendungsgebiet. Bekannt sind beispielsweise der Einsatz als Wärmeübertragungsmittel, als Additiv in Schmierstoffen oder besonders als Ölkomponente in kosmetischen Formulierungen, wobei die beiden letztgenannten Anwendungsgebiete von besonderem Interesse sind.
Diese Anwendungen erfordern jedoch hochreine und qualitativ hochwertige Produkte mit einer ausgezeichneten Farb- und Geruchsqualität, die auch nach längerer Lagerung erhalten bleiben soll.
Vor allem Spuren an Carbonylverbindungen, wie Aldehyde, Ketone, Carbonsäuren oder Lactone, die noch in den Produkten enthalten sind bzw. während der Lagerung entstehen können, beeinträchtigen die Farbe und den Geruch.
Bei den bisher bekannten Verfahren zur Herstellung der Dialkylether kommt es jedoch aufgrund der hohen Reaktionstemperaturen zur Bildung unerwünschter Nebenprodukte, wie Olefine, Carbonylverbindungen, Polymere, welche sowohl den unangenehmen Geruch als auch Beeinträchtigungen der Farbe verursachen.
Aus der Literatur sind verschiedene Varianten bekannt, um Carbonylverbindungen, wie Aldehyde, Ketone, welche schon in geringsten Spuren den Geruch eines Produktes stark beeinflussen können, zu reduzieren. So kann beispielsweise eine Desodorierung, eine Behandlung mit Bleicherde oder der Einsatz unterschiedlichster Reduktionsmittel den Carbonyl-Gehalt absenken. Des weiteren ist es auch möglich, Carbonylverbindungen in Gegenwart von Zinn-Verbindungen zu den entsprechenden Aldol-Kondensaten umzusetzen (Mukaiyama, J. Organomet. Chem., 1990, 382, 39 - 52).

Neben der klassischen Williamson-Ethersynthese werden zur Herstellung der Dialkylether saure Katalysatoren eingesetzt (Ullmann, 5. Aufl., A10, 23 - 34), wie Mineralsäuren, z. B. Schwefelsäure, organische Sulfonsäuren oder saure Feststoffkatalysatoren.
Aus der DE 41 27 230 A1 ist ein Verfahren zur halbkontinuierlichen Herstellung von symmetrischen Dialkylethern bekannt, wobei Alkohole in Gegenwart von Sulfonsäuren als Katalysator bei erhöhter Temperatur dehydratisiert werden, das entstehende Reaktionswasser kontinulerlich abgetrennt und das Rohprodukt nach der Katalysatorabtrennung gereinigt wird.
Um hohe Umsätze an Dialkylethern zu erhalten, muss die Sulfonsäure, beispielsweise Methan-sulfonsäure, Sulfobernsteinsäure, p-Toluolsulfonsäure oder α-Naphthalinsulfonsäure, in Konzentrationen bis zu 5 Gew.-%, bezogen auf die verwendeten Alkohole, eingesetzt werden. Die Sulfonsäuren werden bei der destillativen Reinigung der Dialkylether in Form ihrer Sulfonsäureester teilweise zurückgewonnen bzw. verbleiben im Destillationsrückstand.
Stark eingeschränkt wird dieses Verfahren durch die Siedebereiche der Sulfonsäuren, Alkohole und Dialkylether. Trotz der Destillation der Rohprodukte enthalten diese Dialkylether einen anwendungstechnisch störenden Schwefel-Gehalt, welcher von verbliebenen Katalysatorspuren bzw. deren Zersetzungsprodukten stammt.
Um die Produktqualität hinsichtlich Farbe, Geruch und Lagerstabilität zu verbessern, wird ein Verfahren unter Anwendung üblicher Katalysatoren, wie z.B. Sulfonsäuren oder Schwefelsäure, vorgeschlagen (DE 195 17 049 A1), wonach der hohe Schwefel-Gehalt durch eine zusätzliche und intensive Nachbehandlung mit Natriummethylat bzw. Alkali- oder Erdalkalihydroxid auf Werte von 25 ppm S in den Produkten abgesenkt wird.
Weiterhin sind Verfahren zur Herstellung von Dialkylethern bekannt (DE 19511 668 A1 und DE 19 740 450 A1). Gemäß der in der DE 19511 668 A1 beschriebenen Verfahrensweise werden lineare und/oder verzweigte, gesättigte und/oder ungesättigte, ein- und/oder mehrwertige Alkohole mit 6 bis 22 Kohlenstoffatomen bei erhöhter Temperatur In Gegenwart von Halogenalkansulfonsäuren als Katalysator dehydratisiert, das entstehende Reaktionswasser kontinuierlich abgetrennt und das Rohprodukt nach der Katalysatorabtrennung gereinigt.
Entsprechend den beiden vorgenannten bekannten Verfahren wird die säurekatalysierte Dehydratisierung der Alkohole mit Halogenalkansulfonsäuren allein oder unter Zusatz von Tocopherol bzw. Tocopherolderivaten als Co-Katalysatoren durchgeführt wird. Mit der Wahl dieser stark aciden Halogenalkansulfonsäuren als Veretherungskatalysator können zwar die Katalysatoreinsatzmengen reduziert werden, jedoch verläuft die Dehydratisierung nicht selektiv genug, um die Nebenproduktbildung deutlich zu unterdrücken. So soll erst ein Zusatz von Tocopherol bzw. Tocopherolderivaten in einer Menge bis 5 Gew.-% in der Synthese zu einer Reduzierung der Olefinbildung führen. Weiterhin nachteilig bei beiden Verfahren ist, dass nach der Umsetzung die hochwertige Halogenalkansulfonsäure mit Alkali neutralisiert und als Abwasser entsorgt werden muss.
Der Einsatz eines Co-Katalysators verursacht zudem zusätzliche Kosten.

Bekannt ist auch die Nutzung saurer Feststoffkatalysatoren zur Darstellung der Dialkylether (Park, Catal. Lett., 1997, 46, 1 bis 4). Beschrieben ist der Einsatz von sauren Kationenaustauschern, auf der Basis von Polystyren, Organopolysiloxanen oder vorzugsweise perfluorierten Polymeren. Um kurze Reaktionszeiten zu erzielen, muss aufgrund der geringen Acidität dieser heterogenen Katalysatoren bei der Synthese mit Mengen von bis zu 20 % gearbeitet werden. Das Erreichen eines hohen Umsatzes erfordert hohe Reaktionstemperaturen von 150 bis 250 °C, wodurch der heterogene Katalysator stark geschädigt wird und sich Zersetzungsprodukte bilden. Dadurch wird die Möglichkeit der Abtrennung des Katalysators und dessen Wiedereinsatz erheblich eingeschränkt.
Auch eine mit hohem Aufwand betriebene Regenerierung des abgetrennten Katalysators (SU 170 047) steht einer technischen Verwertung dieses Verfahrens entgegen.

Der Erfindung liegt die Aufgabe zugrunde, ein verbessertes Verfahren zur Herstellung von mittel- und langkettigen hochreinen Dialkylethern zu schaffen, mit einem Katalysator, der nur geringe Katalysatoreinsatzmengen erfordert, sich durch eine hohe Selektivität auszeichnet, einfach aus der Reaktionsmischung abtrennbar ist und ohne Selektivitätsverlust mehrfach wieder verwendet werden kann.

Erfindungsgemäß wird die Aufgabe durch die im Anspruch 1 angegebenen Merkmale gelöst. Geeignete Ausgestaltungsvarianten der Verfahrensweise sind Gegenstand der Ansprüche 2 bis 8. Der Anspruch 9 bezieht sich auf die Verwendung von Zinnhalogenalkansulfonaten als Katalysator zur Dehydratisierung von linearen und/oder verzweigten, gesättigten und/oder ungesättigten, ein- und/oder mehrwertigen Alkoholen mit 6 bis 22 Kohtenstoffatomen zu mittel- und langkettigen Dialkylethern. Durch den Einsatz von Zinnhalogenalkansulfonaten als Katalysator ist es möglich, lineare und/oder verzweigte, gesättigte und/oder ungesättigte, ein- und/oder mehrwertige Alkohole mit 6 bis 22 Kohlenstoffatomen bei erhöhter Temperatur zu hochreinen, mittel- und langkettigen Dialkylethern in hohen Ausbeuten umzusetzen.

Die Umsetzung ist nur mit einer geringen Bildung an Nebenprodukten und deren polymeren Folgeprodukten verbunden. Ein weiterer wesentlicher Vorteil besteht in der einfachen Rückgewinnung des hochwertigen Katalysators in der Aufarbeitungsstufe des Verfahrens und in dem anschließenden mehrmaligen Wiedereinsatz des aufgearbeiteten Katalysators in weiteren Veretherungen.

Als Ausgangsprodukte zur Herstellung der Dialkylether können lineare und/oder verzweigte, gesättigte und/oder ungesättigte, ein- und/oder mehrwertige Alkoholen eingesetzt werden, so dass je nach Wahl des eingesetzten Alkohols symmetrische oder unsymmetrische Dialkylether gebildet werden. Geeignete Ausgangsprodukte sind: Fettalkohole aus der Roelenschen Oxo-Synthese, Ziegler-Alkohole oder bevorzugt native Fettalkohole, wie beispielsweise Capronylalkohol, Önanthalkohol, Caprylalkohol, 2-Ethylhexanol, Pelargonalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol, sowie deren technische Mischungen. Desweiteren können auch Dimer- und Trimeralkohole sowie verzweigte, mehrwertige Polyole, wie TMP, eingesetzt werden.
Die Dehydratisierung der Alkohole zum Dialkylether verläuft in Gegenwart von Zinnhalogenalkansulfonaten. Bei den Halogenalkansulfonsäuren, die in der Zinn-Verbindung als Anion gebunden vorliegen, handelt es sich um organische Sulfonsäuren, mit 1 bis 12 Kohlenstoffatomen und mindestens einem Halogenatom im organischen Rest. Bevorzugt sind vollständig perfluorierte Alkansulfonsäuren, wie Perfluorbutansulfonsäure oder Perfluoroctansulfonsäure.
Als besonders geeigneter Katalysator wird Zinn-(II)-trifluormethansulfonat eingesetzt. Hohe Umsetzungen zum Dialkylether werden hierbei mit Katalysatormengen von 0,01 bis 1 Gew.-%, vorzugsweise 0,05 bis 0,5 Gew.-%, bezogen auf die Alkoholmenge erreicht.

Die Dehydratisierungsreaktion wird bei erhöhter Temperatur durchgeführt. Zur Erzielung hoher Ausbeuten an Dialkylether, verbunden mit ausreichenden Reaktionsgeschwindigkeiten und sehr geringer Nebenproduktbildung, sind Reaktionstemperaturen von 150 °C bis 250 °C zu empfehlen.
Neben der Reaktionstemperatur ist die Katalysatorkonzentration bestimmend für die Reaktionsdauer, welche üblicherweise in einem Bereich von 1 bis 15 Stunden, vorzugsweise 3 bis 7 Stunden liegt.
Es hat sich während der Umsetzung zum Dialkylether als Vorteil erwiesen, das gebildete Reaktionswasser durch Inertgas unterstützt aus dem Reaktionsansatz auszutragen. Der Verlauf bzw. das Ende der Reaktion kann anhand des gebildeten Reaktionswassers oder chromatographisch verfolgt werden.
Abschließend wird der im Reaktionsgemisch verbliebene Katalysator abfiltriert bzw. kann durch eine einfache Wasserwäsche abgetrennt werden. Das so zurückgewonnene Zinnhalogenalkansulfonat kann als Feststoff, meist jedoch in Form wäßriger Lösungen als vollwertiger Katalysator wieder eingesetzt werden.
Die weitere Aufarbeitung des Rohproduktes kann je nach dem gewünschten Verwendungszweck des Dialkylethers durch Waschen bzw. destillativ erfolgen.

### Beispiel 1: Herstellung von Di-n-octyl-ether

Zur Herstellung des Dioctylethers wird ein 1I-Vierhalsrundkolben eingesetzt, welcher mit einer dosierbaren N₂-Einleitung, einem KPG-Rührer und mit einem Wasserabscheider ausgestattet ist. Zur Kondensation der gasförmigen Reaktionskomponenten schließt sich an den Wasserabscheider ein Rückflußkühler an. Nach der Phasentrennung des kondensierten Octanol/Wasser-Gemisches fließt die organische Phase stetig in den Reaktionsansatz zurück.
Zu 520 g (4 mol) n-Octanol (n-Octanol 99,1 %; OHZ 431) werden unter Rühren und getauchter N₂-Einleitung (2 I N₂/h) bei Raumtemperatur als Katalysator 750 mg Zinn-(II)-trifluormethansulfonat (0,15 Gew.-% bezogen auf Octanol) hinzugefügt und der Reaktionsansatz auf eine Temperatur von 195 °C erwärmt. Kurz unterhalb des Siedepunktes vom n-Octanol setzt die Dehydratisierung ein. Der Verlauf der Reaktion wird gaschromatographisch verfolgt. Ab einem Umsatzgrad von ca. 70 % erhöht man die Reaktionstemperatur langsam auf 220 °C und behält diese bis zum Ende der Reaktion bei. Mit dem Erreichen von ca. 90 - 93 % Dioctylether im Reaktionsgemisch wird die Reaktion abgebrochen und der Ansatz wird unter Rühren und N₂-Einleitung auf 90 °C abgekühlt. Anschließend wird das Rohprodukt dreimal mit je 15 ml destillierten Wasser unter N₂-Atmosphäre gewaschen.
Die gesammelten Waschwässer enthalten den Katalysator und werden als wäßrige Lösung bei weiteren Synthesen wiedereingesetzt.
Das getrocknete Rohprodukt wird im Vakuum über eine Kolonne destillativ gereinigt, Spuren Octanol werden mit Wasserdampf aus dem Endprodukt entfernt.
Es wurde eine Ausbeute von 439 g (90,7 % d.Th.) an Di-n-octylether erhalten.
Gehalt an Di-n-octyl-ether: 99,5 % (GC);
APHA 2
OHZ: 0,5
VZ: 0,2
Peroxid-Gehalt: 0,05 mvalO₂/kg
Carbonyl-Gehalt: < 5 ppm
Schwefel-Gehalt: < 1 ppm

### Beispiel 2: Herstellung von Di-n-myristyl-ether

In der gleichen Apparatur analog dem Beispiel 1 werden bei einer Temperatur von 50 °C unter Stickstoff 645 g (3 mol) Myristylalkohol (Myristylalkohol 99,1 %; OHZ 261) und als Katalysator 645 mg Zinn-II-triflat (0,1 Gew.-% bezogen auf Myristylalkohol) unter Rühren vorgelegt und auf eine Temperatur von 200 °C erwärmt. Mit der Beendigung der Wasserabscheidung, d.h. einem Gehait an ca. 90 % Dimyristylether im Reaktionsgemisch wird die Reaktion abgebrochen. Anschließend wird das Rohprodukt dreimal mit je 25 ml destillierten Wasser bei 80 °C unter N₂-Atmosphäre gewaschen und so der Katalysator abgetrennt.
Die gesammelten Waschwässer enthalten den Katalysator, welcher als wäßrige Lösung bei weiteren Synthesen wiedereingesetzt werden kann.
Das getrocknete Rohprodukt wird im Vakuum über eine gut trennende Kolonne destillativ gereinigt, wobei der Dimyristylether als Hauptlauf (205 bis 207 °C/1mbar) abgetrennt wird.

Unter diesen Bedingungen wurde eine Ausbeute von 559 g (90,4 % d.Th.) an Dimyristylether erzielt.
Gehalt an Di-n-myristyl-ether: 98,9 % (GC);
APHA 23
OHZ: 0,8
VZ: 0,4
Peroxid-Gehalt: 0,1 mvalO₂/kg
Carbonyl-Gehalt: 15 ppm
Schwefel-Gehalt: < 1 ppm
mp. 43 - 45 °C

### Beispiel 3: Herstellung von Di-(2-Octyl-1-dodecyl)-ether

Zu 618 g (2 mol) technischen 2-Octyl-1-dodecanol (GC 98,,5 %; OHZ 181,5) werden bei 60 °C als Katalysator 432 mg Zinn-(II)-trifluormethansulfonat (0,07 Gew.-% bezogen auf den verzweigten Alkohol) unter Rühren und getauchter N₂-Einleitung (2 l N₂/h) hinzugefügt und der Reaktionsansatz auf eine Temperatur von 180 °C erwärmt. Nach der Abtrennung von 18 ml Reaktionswasser wird die Dehydratisierung durch ein Abkühlen auf eine Temperatur von 60 °C beendet und der Katalysator unter Zusatz von Celite als Filterhilfsmittel vom Reaktionsprodukt abfiltriert. Unter Vakuum wurden nicht umgesetzter Alkohol und gebildete Olefine destillativ abgetrennt und abschließend das als Sumpf anfallende Produkt mit Bleicherde (Tonsil 411) aufgehellt. Es wurde ein klares Öl in einer Ausbeute von 521 g (86,8 % d.Th.) erhalten.
OHZ 1,4
VZ 0,9
IZ 1,8
APHA 68
Peroxid-Gehalt: 0,8 mvalO₂/kg
Carbonyl-Gehalt: 23 ppm
Schwefel-Gehalt: <1 ppm

### Beispiel 4: Herstellung von Di-n-octylether unter Wiederverwendung der abgetrennten Katalysatorlösung gemäß Beispiel 1

Analog dem Beispiel 1 werden in der gleichen Apparatur bei Raumtemperatur 520 g (4 mol) n-Octanol sowie die abgetrennte wäßrige Katalysator-Lösung (ca. 45 ml) gemäß dem Beispiel 1 unter Rühren und getauchter N₂-Einleitung (2 l N₂/h) auf 110 °C erwärmt.
Nach der Abtrennung von ca. 45 ml Wasser (aus der wäßrigen Katalysator-Lösung) erfolgt die Dehydratisierung und die Aufarbeitung in analoger Weise wie im Beispiel 1. Die abgetrennte wäßrige Katalysatorlösung wird wieder zur Herstellung von Di-n-octylether unter den gleichen Bedingungen eingesetzt (Beispiel 4a). Nachfolgend werden zwei weitere Versuche (Beispiele 4b und 4c) unter gleichen Bedingungen durchgeführt, wobei im Beispiel 4b die in Beispiel 4a abgetrennte Katalysatorlösung eingesetzt wird und im Beispiel 4c die in Beispiel 4b abgetrennte Katalysatorlösung. Die Qualität des abgetrennten Katalysators wurde vor dem jeweiligen Wiedereinsatz überprüft, wobei in der nachfolgenden Tabelle die ermittelten Ergebnisse angegebenen sind.

| Wiedereinsatz des abgetrennten Katalysators | Ausbeute an Dioctylether (% d.Th.) | Reinheit Dioctylether (Gew.-%) | APHA | Peroxid-Gehalt (mvalO₂/kg) | Carbonyl-Gehalt (ppm) |
|---|---|---|---|---|---|
| | | | | | |
| Beispiel 4 | 88,5 | 99,1 | 3 | 0,05 | < 5 |
| Beispiel 4a | 90,2 | 99,2 | 5 | 0,1 | 8 |
| Beispiel 4b | 89,6 | 98,8 | 2 | 0,08 | < 5 |
| Beispiel 4c | 91,5 | 99,4 | 3 | 0,03 | < 5 |

Wie die vorliegenden Ergebnisse zeigen, führte der mehrmalige Einsatz des Katalysators zu keinen Selektivitätsverlusten.

## Patentansprüche

1. Verfahren zur Herstellung von mittel- und langkettigen Dialkylethern, bei dem lineare und/oder verzweigte, gesättigte und/oder ungesättigte, ein- und/oder mehrwertige Alkohole mit 6 bis 22 Kohlenstoffatomen bei erhöhter Temperatur in Gegenwart eines Katalysators dehydratisiert werden, das entstehende Reaktionswasser kontinuierlich abgetrennt und das Rohprodukt nach der Katalysatorabtrennung gereinigt wird, **dadurch gekennzeichnet, dass** als Katalysator Zinnhalogenalkansulfonate eingesetzt werden.

2. Verfahren nach Anspruch 1, **dadurch gekennzeichnet, dass** als Zinnhalogenalkansulfonat Zinn-(II)-trifluormethansulfonat eingesetzt wird.

3. Verfahren nach einem der Ansprüche 1 oder 2, **dadurch gekennzeichnet, dass** das Zinnhalogenalkansulfonat in einer Menge von 0,01 bis 1 Gew.-%, bezogen auf die Alkoholmenge, eingesetzt wird.

4. Verfahren nach einem der Ansprüche 1 bis 3, **dadurch gekennzeichnet, dass** das Zinnhalogenalkansulfonat in einer Menge von 0,05 bis 0,5 Gew.-%, bezogen auf die Alkoholmenge, eingesetzt wird.

5. Verfahren nach einem der Ansprüche 1 bis 4, **dadurch gekennzeichnet, dass** die Umsetzung bei einer Temperatur von 150 °C bis 250 °C durchgeführt wird.

6. Verfahren nach einem der Ansprüche 1 bis 5, **dadurch gekennzeichnet, dass** synthetische Alkohole, Alkohole auf nativer Basis und/oder deren technische Gemische eingesetzt werden.

7. Verfahren nach einem der Ansprüche 1 bis 6, **dadurch gekennzeichnet, dass** das Zinnhalogenalkansulfonat als Feststoff oder als wässrige Phase abgetrennt und als Katalysator ein- oder mehrmals wiederverwendet wird.

8. Verfahren nach einem der Ansprüche 1 bis 7, **dadurch gekennzeichnet, dass** das während der Umsetzung gebildete Reaktionswasser durch Inertgas unterstützt aus dem Reaktionsansatz ausgetragen wird.

9. Verwendung von Zinnhalogenalkansulfonaten als Katalysator zur Dehydratisierung von linearen und/oder verzweigten, gesättigten und/oder ungesättigten, ein- und/oder mehrwertigen Alkoholen mit 6 bis 22 Kohlenstoffatomen zu mittel- und iangkettigen Dialkylethern.

## Claims

1. Method for the preparation of medium-chain and long-chain dialkyl ethers in which linear and/or branched-chain saturated and/or unsaturated monohydric and/or polyhydric alcohols with 6 to 22 carbon atoms are dehydrated at elevated temperature, in the presence of a catalyst, with the water formed by the reaction being continuously separated and the raw product purified after the catalyst is removed, **characterized in that** tin-halide alkane sulphonates are used as catalyst.

2. Method according to claim 1 **characterized in that** tin(II)-trifluoromethane sulphonate is used as a tin-halide alkane sulphonate compound.

3. Method according to one of the claims 1 or 2 **characterized in that** the tin-halide alkane sulphonate is added in a quantity of 0,01 to 1 percent in weight, in relation to the amount of alcohol present.

4. Method according to one of the claims 1 to 3 **characterized in that** the tin-halide alkane sulphonate is added in a quantity of 0,05 to 0,5 percent in weight, in relation to the amount of alcohol present.

5. Method according to one of the claims 1 to 4 **characterized in that** the transformation process takes place at temperatures from 150 °C to 250 °C.

6. Method according to one of the claims 1 to 5 **characterized in that** synthetic alcohols, native alcohols and/or their commercial mixtures are used.

7. Method according to one of the claims 1 to 6 **characterized in that** the tin-halide alkane sulphonate is separated as solid or aqueous phase to be re-used as a catalyst once or several times.

8. Method according to one of the claims 1 to 7 **characterized in that** the water formed in the transformation process is stripped from the reaction mixture using inert gas as a stripping agent.

9. Utilization of tin-halide alkane sulphonates as catalyst for the dehydration of linear and/or branched-chain saturated and/or unsaturated monohydric and/or polyhydric alcohols with 6 to 22 carbon atoms to be transformed into medium-chain and long-chain dialkyl ethers.

## Revendications

1. Procédé pour la préparation d'éthers de dialkyle à chaîne moyenne et à chaîne longue pour lequel des monoalcools et/ou polyalcools linéaires et/ou ramifiés, saturés et/ou insaturés comprenant 6 à 22 atomes de carbone sont déshydratés à une température élevée en présence d'un catalyseur, l'eau produite pendant la réaction est séparée en continu et le produit brut est épuré après la séparation du catalyseur, **caractérisé par le fait que** l'on utilise des alcanesulfonates d'halogénure d'étain comme catalyseur.

2. Procédé selon la revendication 1, **caractérisé en ce que** l'on utilise du trifluorométhanesulfonate d'étain (II) comme alcanesulfonate d'halogénure d'étain.

3. Procédé selon l'une quelconque des revendications 1 ou 2, **caractérisé en ce que** l'alcanesulfonate d'halogénure d'étain est employé dans une quantité comprise entre 0,01 et 1 % en poids par rapport à la quantité d'alcool.

4. Procédé selon l'une quelconque des revendications 1 à 3, **caractérisé en ce que** l'alcanesulfonate d'halogénure d'étain est employé dans une quantité comprise entre 0,05 et 0,5 % en poids par rapport à la quantité d'alcool.

5. Procédé selon l'une quelconque des revendications 1 à 4, **caractérisé en ce que** la transformation est réalisée à une température comprise entre 150 °C et 250 °C.

6. Procédé selon l'une quelconque des revendications 1 à 5, **caractérisé en ce que** l'on utilise des alcools synthétiques, des alcools naturels et/ou leurs mélanges techniques.

7. Procédé selon l'une quelconque des revendications 1 à 6, **caractérisé en ce que** l'alcanesulfonate d'halogénure d'étain est séparé en tant que solide ou en tant que phase aqueuse et qu'il est réutilisé comme catalyseur une fois ou plusieurs fois.

8. Procédé selon l'une quelconque des revendications 1 à 7, **caractérisé en ce que** l'eau de la réaction s'étant formée au cours de la transformation est évacuée de la préparation de la réaction avec le soutien d'un gaz inerte.

9. Utilisation d'alcanesulfonates d'halogénure d'étain comme catalyseur pour la déshydratation de monoalcools et/ou polyalcools linéaires et/ou ramifiés, saturés et/ou insaturés comprenant 6 à 22 atomes de carbone en éthers de dialkyle à chaîne moyenne et à chaîne longue.
